# EUROPEAN PATENT SPECIFICATION

(11) **EP 2 484 320 B1**
(45) Date of publication and mention of the grant of the patent: **05.11.2014**
(21) Application number: 10818640.4
(22) Date of filing: 13.08.2010
(51) Int. Cl.: A61F 13/49, A61F 13/15, A61F 13/511

(54) **DISPOSABLE WEARING ARTICLE CAPABLE OF ABSORBING BODILY FLUIDS**
EINWEGKLEIDUNGSSTÜCK ZUR ABSORPTION VON KÖRPERFLÜSSIGKEITEN
ARTICLE À PORTER JETABLE ABSORBANT LES FLUIDES ORGANIQUES

(30) Priority: 28.09.2009 JP 2009223445
(43) Date of publication of application: 08.08.2012
(73) Proprietor: Unicharm Corporation, Ehime 799-0111 (JP)
(72) Inventor: YAGO, Toshiya, Kanonji-shi Kagawa 769-1602 (JP); OHASHI, Naoto, Kanonji-shi Kagawa 769-1602 (JP); WAKASUGI, Kei, Kanonji-shi Kagawa 769-1602 (JP); YAMANAKA, Yasuhiro, Kanonji-shi Kagawa 769-1602 (JP)
(74) Representative: Knights, Rupert
(86) International application number: PCT/JP2010/063764
(87) International publication number: WO 2011/036964

(56) References cited:
- EP-A1- 2 034 067
- EP-A2- 0 958 802
- EP-A2- 0 962 208
- JP-A- 5 269 168
- JP-A- 58 132 155
- JP-A- 2002 524 664
- JP-A- 2008 025 079
- JP-A- 2009 030 218
- JP-A- 2009 118 920
- JP-A- 2009 118 921
- US-B1- 6 171 682

## Description

### {Technical Field}

This invention relates to disposable bodily fluid absorbent wearing articles such as disposable diapers and sanitary napkins.

### {Background}

Conventionally, disposable bodily fluid absorbent wearing articles such as disposable diapers and sanitary napkins including a core material assembly interposed between a liquid-pervious topsheet and a liquid-impervious backsheet are known. It is also known to form the topsheet with ridges and troughs extending in parallel to each other in one direction and arranged alternately in a direction being orthogonal to the direction so that these ridges and troughs face the wearer's skin.

For example, JP S58-132155 A (PTL 1) discloses a liquid-pervious nonwoven fabric suitable to be used as a surface material of disposable diapers or sanitary napkins. This nonwoven fabric has a convexo-concave pattern composed of ridges and troughs extending in parallel to each other in a machine direction in manufacturing process for the nonwoven fabric wherein the fiber density is relatively low in the ridges and relatively high in the troughs.

JP 2008-25079 A (PTL 2) also discloses a liquid-pervious nonwoven fabric suitable to be used as a surface material of disposable diapers or sanitary napkins. In this nonwoven fabric, one of both surfaces is formed to be substantially flat and the other surface is formed with ridges and troughs extending in parallel to each other in the machine direction in such a manner that these ridges and troughs are arranged alternately in a direction orthogonal to the machine direction. The ridges have a density higher than that of the troughs.

JP 2009-030218 A (PTL 3) also discloses a liquid-pervious nonwoven fabric suitable to be used as a topsheet of disposable diapers or sanitary napkins. In this nonwoven fabric also, one of both surfaces is formed to be substantially flat and the other surface is formed with ridges and troughs extending in parallel to each other in the machine direction and are arranged alternately in a direction orthogonal to the machine direction. The ridges contain fibers oriented in the thickness direction of the nonwoven fabric.

### {Citation List}

### {Patent Literature}

{PTL 1} JP S58-132155 A
{PTL 2} JP 2008-25079 A
{PTL 3} JP 2009-030218 A

Further prior art arrangements are known from EP 0962208, US 6171682, EP 0958802 and EP 2034067.

### {Summary}

### {Technical Problem}

In the bodily fluid absorbent wearing articles such as disposable diapers and sanitary napkins, opposite side edges of the article are provided with the leakage-barriers in order to prevent bodily fluids from leaking out beyond these side edges and, between the opposite leakage-barriers, the bodily fluid absorbent core material assembly is sandwiched between the liquid-pervious topsheet and the liquid-impervious backsheet. When the topsheet disclosed in any one of PTL 1 through 3 is used, it is possible to ensure a sufficient volume of air-permeable clearances between the article wearer's skin and the topsheet and, in contrast with the case in which the topsheet of which both surfaces are flat, correspondingly the contact area between the skin and the topsheet may be reduced. However, when it is tried to bond the proximal side edges of the respective leakage-barriers formed of the belt-like sheet strips to the topsheet, it is easy to bond the proximal side edges to the ridges of the nonwoven fabric but it is not necessarily easy to bond the proximal side edges to the troughs. To overcome such inconvenience, it will be required to enlarge the dimension of the proximal side edges in the width direction compared to the case in which the proximal side edges are bonded to the flat surface of the nonwoven fabric.

An object of the present invention is to improve the conventional disposable bodily fluid absorbent wearing article so that leakage-barriers may be easily attached to the topsheet being formed with the ridges and the troughs extending in parallel to each other.

### {Solution to Problem}

### This invention provides a disposable bodily fluid absorbent wearing article as recited by Claim 1.

In such a wearing article, this invention resides in that the topsheet extends outward in the width direction at least partially beyond opposite side edges of the core material assembly to define lateral regions extending outside the core material assembly and, in these lateral regions, the ridges are in a compressed state to become lower in height.

According to one embodiment of this invention, the topsheet is in a compressed state also in part of a central region defined inboard of the lateral regions in the width direction to become lower in height than the ridges.

According to further another embodiment of this invention, the wearing article is a sanitary napkin.

According to yet another embodiment of this invention, the wearing article is a disposable diaper including a crotch region extending in the front-back direction, a front waist region lying in front of the crotch region and a rear waist region lying behind the crotch region and the core material assembly is contained at least in the crotch region of these regions.

According to an alternative embodiment of this invention, the lateral regions of the topsheet extending in the width direction beyond the opposite side edges of the core material assembly are overlapped and bonded together with the backsheet and the respective ones of the opposite side edges of the belt-like sheet strips forming the respective leakage-barriers are overlapped and bonded together outside the respective lateral regions of the topsheet as viewed in the width direction.

According to another alternative embodiment of this invention, the ridges contain the short fibers oriented in a height direction of the ridges.

### {Advantageous Effects of Invention}

In the disposable bodily fluid absorbent wearing article according to this invention including the topsheet formed on its outer surface with the ridges and the troughs alternately arranged, the opposite lateral regions of the topsheet extending outward at least partially beyond the opposite side edges of the bodily fluid absorbent core material assembly are in a state such that the ridges have been compressed and the thickness of the topsheet has been reduced. In such a topsheet, the opposite lateral regions are sufficiently flattened to assure that the proximal side edges of the respective leakage-barriers may be easily bonded to the opposite lateral regions of the topsheet.

### {Brief Description of Drawings}

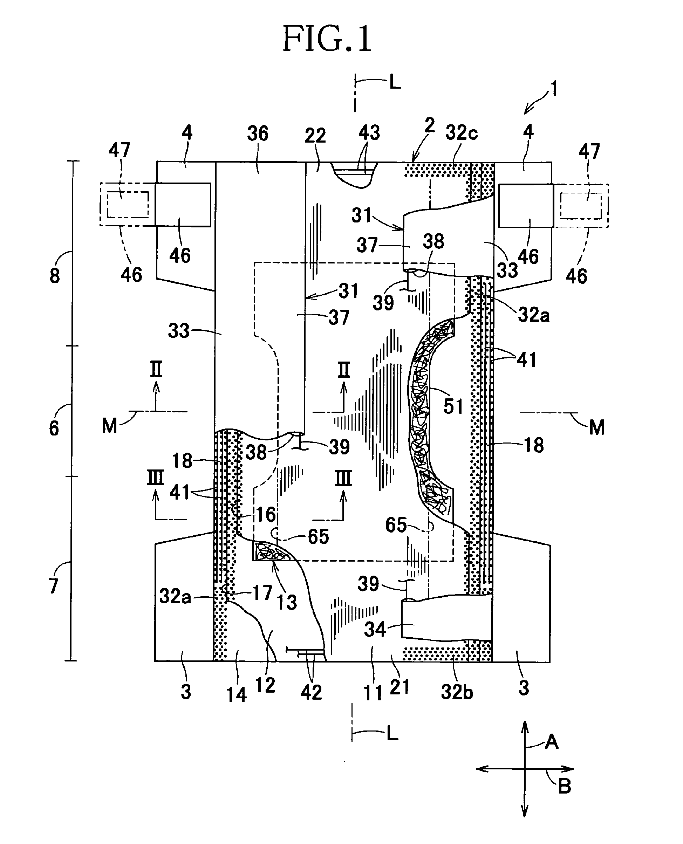

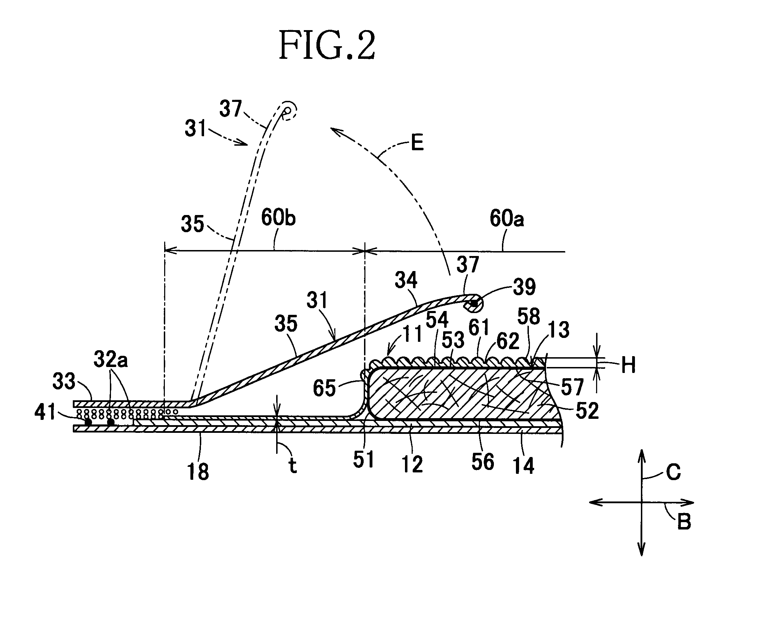

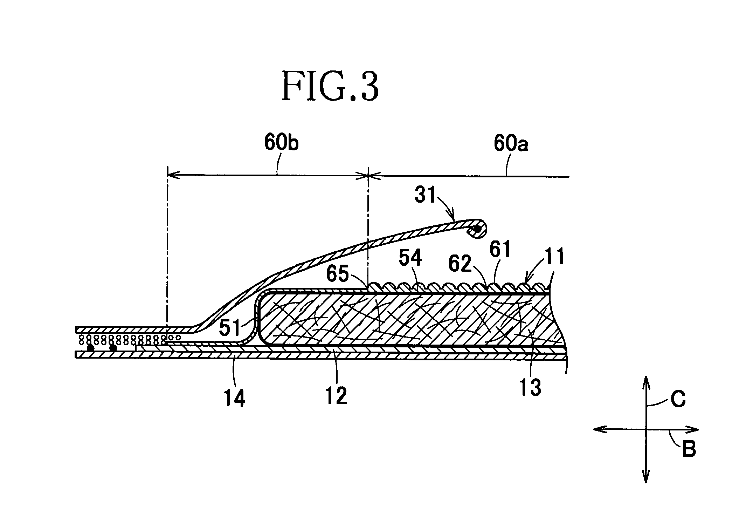

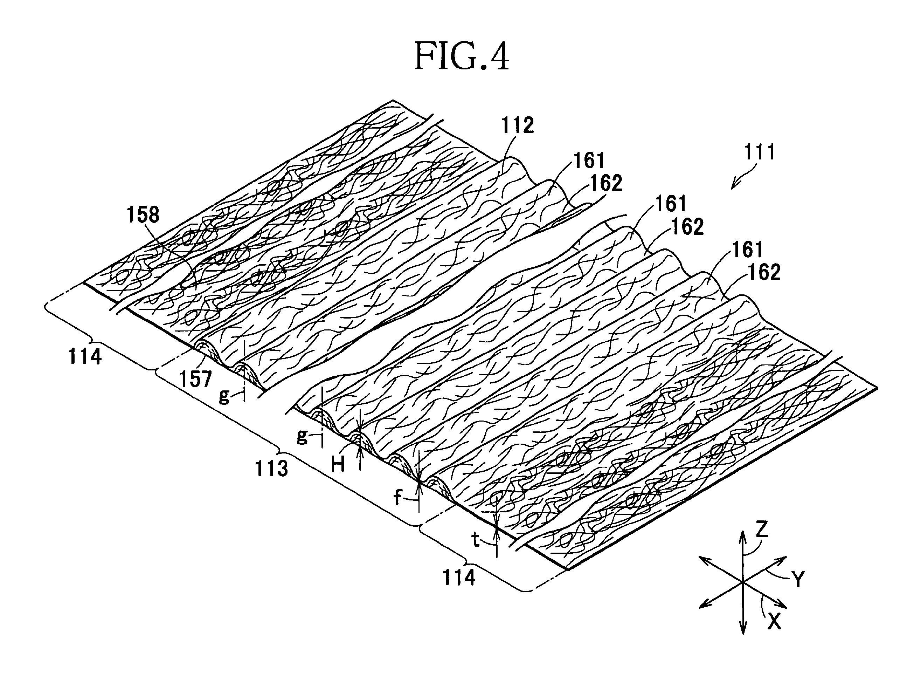

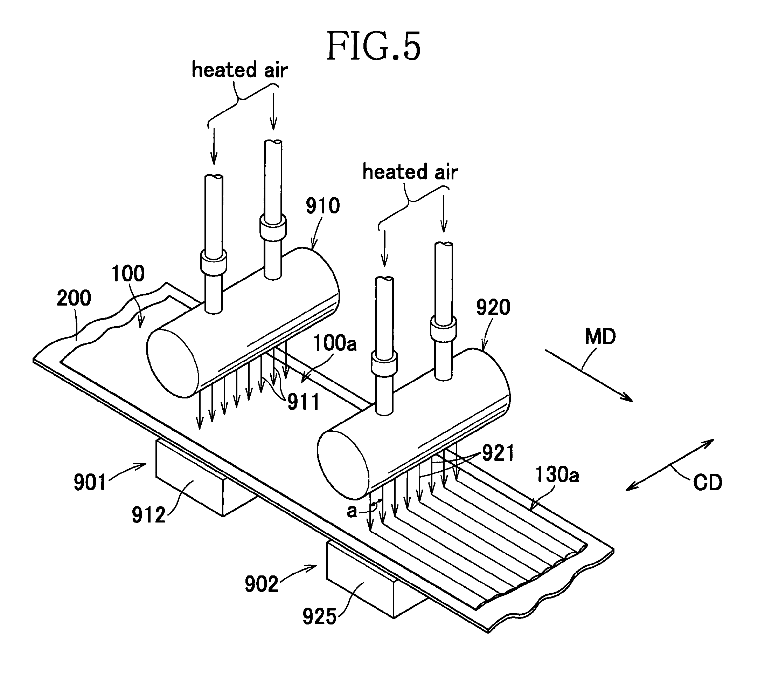

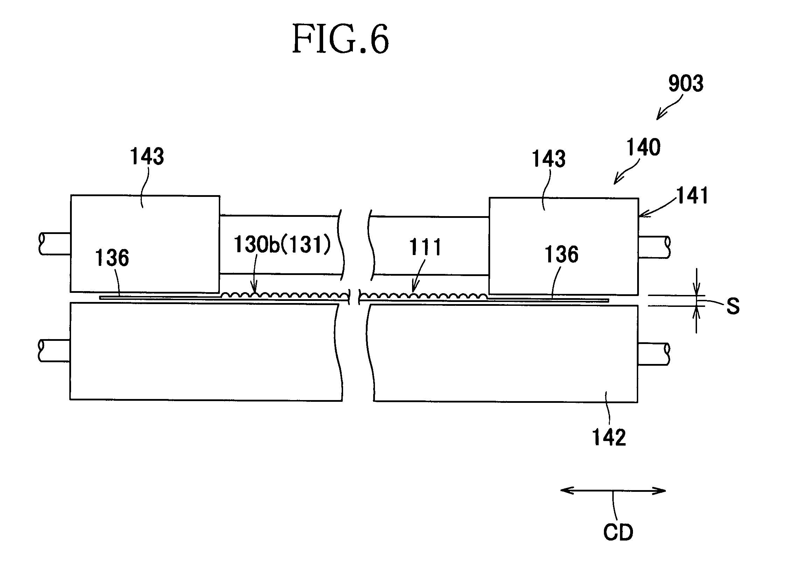

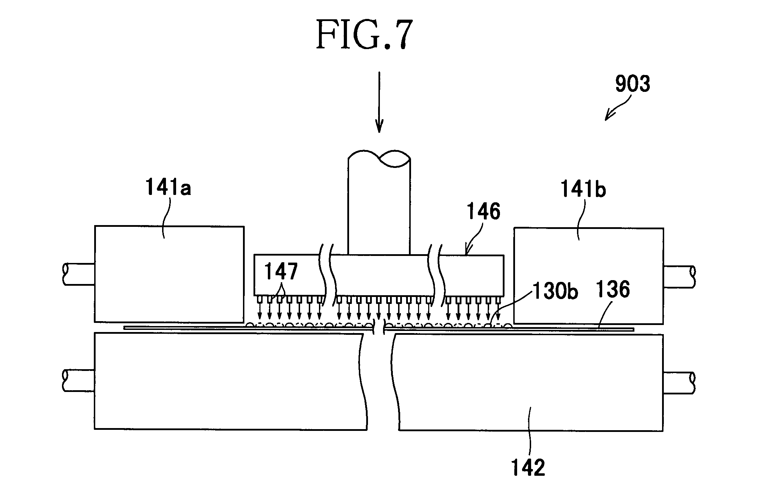

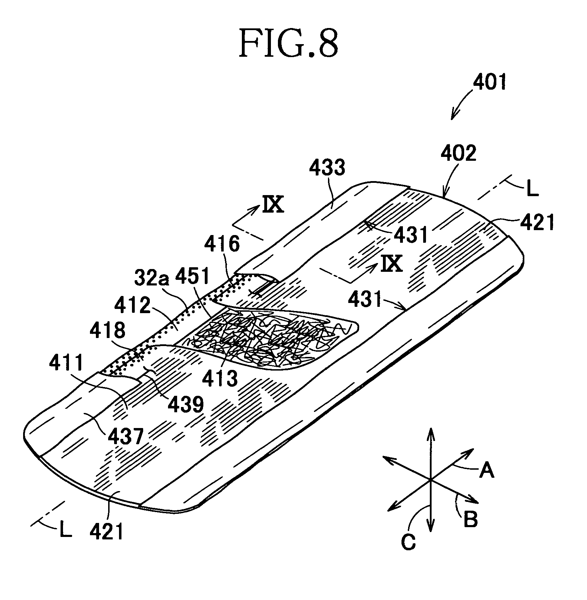

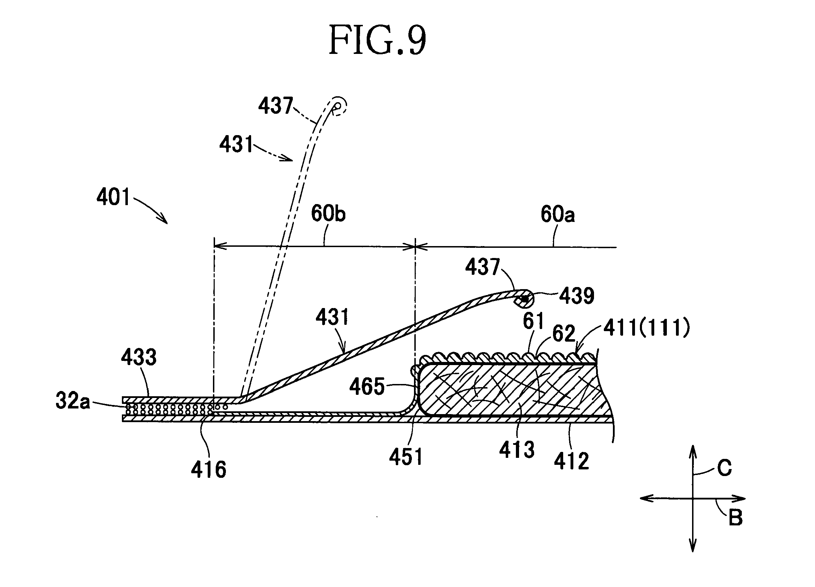

{Fig. 1} Fig. 1 is a partially cutaway plan view of a diaper.
{Fig. 2} Fig. 2 is a sectional view taken along line II-II in Fig. 1.
{Fig. 3} Fig. 3 is a sectional view taken along line III-III in Fig. 1.
{Fig. 4} Fig. 4 is a partially cutaway plan view of an individually cut out sheet defining a topsheet.
{Fig. 5} Fig. 5 is adiagrampartially illustratingamanufacturing process for the individually cut out sheet.
{Fig. 6} Fig. 6 is adiagrampartially illustrating amanufacturing process for the individually cut out sheet.
{Fig. 7} Fig. 7 is a diagram similar to Fig. 6, illustrating one embodiment of the manufacturing process.
{Fig. 8} Fig. 8 is a partially cutaway perspective view of a sanitary napkin.
{Fig. 9} Fig. 9 is a sectional view taken along a line IX-IX in Fig. 8.

### {Description of Embodiments}

Details of a disposable bodily fluid absorbent wearing article according to this invention will be described hereunder with reference to the accompanying drawings.

Fig. 1 is a partially cutaway plan view of an open-type disposable diaper 1 as an example of the wearing article according to this invention. The diaper 1 has a front-back direction A and a width direction B being orthogonal to each other, and includes a rectangular chassis 2 shaped to be relatively long in the front-back direction A, a pair of front wings 3 attached to a front section of the chassis 2 so as to extend in the width direction B and a pair of rear wings 4 attached to a rear section of the chassis 2 so as to extend in the width direction B. In the front-back direction A of the chassis 2, a crotch region 6 is defined between the front wings 3 and the rear wings 4, a front waist region 7 is defined in front side of the crotch region 6 and a rear waist region 8 is defined in rear side of the crotch region 6.

The chassis 2 includes a liquid-pervious topsheet 11, a liquid-impervious backsheet 12 and a bodily fluid absorbent core material assembly 13 interposed between these two sheets 11, 12 wherein the backsheet 12 is covered with an outer sheet 14 made of a nonwoven fabric having a comfortable texture. The topsheet 11 and the backsheet 12 extend outward beyond a peripheral regions 51 of the core material assembly 13 and are overlapped together outside the peripheral regions 51 and bonded to each other with hot melt adhesive (not shown). While the topsheet 11 and the backsheet 12 exemplarily illustrated are dimensioned to be the same in the front-back direction A, in the width direction B, the backsheet 12 extends outward beyond opposite side edges 16 of the topsheet 11. While the outer sheet 14 is dimensioned to be the same as the backsheet 12 in the front-back direction A, in the width direction B, the outer sheet 14 extends beyond opposite side edges 17 of the backsheet 12. Respective outer extensions of these topsheet 11, backsheet 12 and outer sheet 14 beyond the core material assembly 13 define opposite side edges 18 and front and rear ends 21, 22 of the chassis 2. Along the opposite side edges 18, paired leakage-barriers 31 are formed of individually cut out sheets which are relatively long in the front-back direction A. These leakage-barriers 31 respectively include proximal side edges 33 bonded to the side edges 18 with hot melt adhesive 32a, front ends 34 bonded to the front end 21 with hot melt adhesive 32b, rear ends 36 bonded to the rear end 22 with hot melt adhesive 32c, and free side edges 37 lying on the inner side of the chassis 2 compared to the proximal side edges 33 and separably overlapping the topsheet 11 wherein the respective free side edges 37 form sleeves 38 and elastic members 39 are attached under tension to inner sides of the respective sleeves 38 with hot melt adhesives (not shown).

Along the opposite side edges 18 of the chassis 2, leg elastic members 41 are disposed between the outer sheet 14 and the proximal side edges 33 of the respective leakage-barriers 31 and attached under tension in the front-back direction A to the outer sheet 14 with hot melt adhesives (not shown). Along the front end 21 of the chassis 2, front waist elastic members 42 are disposed between the topsheet 11 and the backsheet 12 and bonded under tension in the width direction B to at least one of these sheets 11, 12 with hot melt adhesive (not shown). Along the rear end 22 of the chassis 2, rear waist elastic members 43 are disposed between the topsheet 11 and the backsheet 12 and bonded under tension in the width direction B to at least one of these two sheets 11, 12 with hot melt adhesives (not shown) .

The chassis 2 formed in the manner as described above is provided on the opposite side edges 18 in the front waist region 7 with the front wings 3 extending outward in the width direction B and on the opposite side edges 18 in the rear waist region 8 with the rear wings 4 extending outward in the width direction B. The rear wings 4 respectively have tape fasteners 46 attached thereto. When it is desired to put the diaper 1 on the wearer's body, the tape fasteners 46 may be extended outward in the width direction B as indicated by imaginary lines and may be releasably fastened to the outer surface of the chassis 2 or to the outer surface of the front winds 3 with pressure-sensitive adhesive 47 applied to the inner surfaces of the respective tape fasteners 46.

The diaper 1 having such chassis 2 is symmetric about a center line L bisecting the width of the chassis 2 and when the chassis 2 is bowed in the front-back direction A with the topsheet 11 inside to be U-shaped, the free side edges 37 of the leakage-barriers 31 are spaced apart from the topsheet 11 under contraction of the elastic members 39 and the leakage-barriers 31 rise on the topsheet 11 as will be illustrated later in Fig. 2. The leakage-barriers 31 standing up in this manner function to prevent bodily fluids flowing on the topsheet 11 of the crotch region 6 in the width direction B from leaking out of the diaper 1.

Fig. 2 is an enlarged sectional view taken along line II-II in Fig. 1 wherein a thickness direction of the diaper 1 is indicated by a double-headed arrow C. Referring to Fig. 2, the core material assembly 13 occupies at least a midsection in the width direction B of the crotch region 6 and including water-absorptive material 52 such as fluff wood pulp and superabsorbent polymer particles wrapped with a wrapping sheet 53 formed of, for example, tissue paper or a liquid-pervious nonwoven fabric which is liquid-pervious, preferably not only liquid-pervious but also liquid-absorptive, and even more preferably, in addition to these two properties, liquid-dispersant. The core material assembly 13 has an upper surface 54 covered with the topsheet 11 and a lower surface 56 covered with the backsheet 12. Along the opposite side edges 18 of the chassis 2 defined by the topsheet 11, the backsheet 12 and the outer sheet 14, the proximal side edges 33 of the respective leakage-barriers 31 are bonded to the topsheet 11, the backsheet 12 and the outer sheet 14 opposed to the respective proximal side edges 33 with hot melt adhesive 32a. The situation in which the free side edges 37 of the respective leakage-barriers 31 and intermediate portions 35 defined between the free side edges 37 and the proximal side edges 33, respectively, are standing up on the topsheet 11 is indicated by imaginary lines.

Referring again to Fig. 2, the topsheet 11 has an inner surface 57 facing the core material assembly 13 and an outer surface 58 opposite to the inner surface 57 wherein the inner surface 57 is substantially flat. A central region 60a of the outer surface 58 defined inboard of peripheral regions 51 of the core material assembly 13 in the crotch region 6 is formed with ridges 61 and troughs 62 alternating in the width direction B and lateral regions 60b of the outer surface 58 extending outward from the peripheral regions 51 are flat except regions in contact with the peripheral regions 51. A thickness t of the topsheet 11 in the lateral regions 60b is smaller than a height H of the ridges 61 in the central region 60a. The proximal side edges 33 of the respective leakage-barriers 31 are bonded to the topsheet 11 in the flat lateral regions 60b thereof. With the diaper put on the wearer's body, the central region 60a of the topsheet 11 may easily come in contact with the wearer's skin at the respective ridges 61 but not easily come in contact with the wearer's skin at the respective troughs 62 and, in consequence, air passages may be formed between the wearer's skin and the topsheet 11 along the respective troughs 62 extending in the front-back direction A. Boundary regions 65 between the central region 60a and the respective lateral regions 60b in Fig. 2 are indicated by imaginary lines.

Fig. 3 is a sectional view taken along line III-III in Fig. 1. The core material assembly 13 partially hidden behind the topsheet 11 as indicated by dotted line in Fig. 1 has a known hourglass-shape having its width narrowest in the crotch region 6 and gradually enlarged toward the front waist region 7 and the rear waist region 8. The line III-III extends across the core material assembly 13 in its region having an enlarged width. The boundary regions 65 between the central region 60a and the lateral regions 60b rectilinearly extend in parallel to the center line L (See Fig. 1) in the front-back direction A. In Fig. 2, these boundary regions 65 lie adjacent to the peripheral regions 51 but in Fig. 3, the positions of the peripheral regions 51 are shifted outward in the width direction B and, in consequence, the respective boundary regions 65 are spaced from the associated peripheral regions 51. As will be apparent from Figs. 2 and 3, the flat lateral regions 60b of the topsheet 11 are defined in at least portions of the peripheral regions 51 of the core material assembly 13 extending in the front-back direction A, more specifically, defined outboard of a portion of the peripheral regions 51 of the core material assembly 13 having a narrowest width.

Fig. 4 is a partially cutaway perspective view of a sheet 111 to be used as the topsheet 11. The sheet 111 is a nonwoven fabric formed of short fibers 112 of thermoplastic synthetic resin having a fineness in a range of 1 to 8dtex and previously treated to become hydrophilic wherein these short fibers 112 are interlaced and fused together. This sheet 111 has an upper surface 158 and a lower surface 157 and has a mass per unit area in a range of 10 to 60g/m². The short fibers 112 have a fiber length in a range of 5 to 80mm and the fiber length of the short fibers 112 is uniform or different as the case may be. Furthermore, the short fibers 112 are straight or mechanically or thermally crimped as the case may be. As the thermoplastic synthetic resin forming the short fibers 112, for example, polyethylene, polypropylene, nylon or polyester may be used. As the short fibers 112, it is possible to use core-in-sheath type or side-by-side type conjugate fibers made from at least two of the aforementioned various kinds of thermoplastic synthetic resins. Central region in a first direction X of the sheet 111 corresponding to the width direction B of the diaper 1 in Fig. 1 is formed with an undulating region 113 and, on both sides of the undulating region 113, flat regions 114 are formed. The undulating region 113 is formed on its upper surface 158 with ridges 161 and troughs 162 alternating in the first direction X at a pitch in a range of 2 to 7mm. These ridges 161 and troughs 162 extend in parallel to each other in a second direction Y corresponding to the front-back direction A of the diaper 1. A dimension H in a third direction Z orthogonal to the first direction X and the second direction Y between an upper surface 158 and a lower surface 157 of the ridges 161 corresponds to a height of the ridges 161. In this invention, this dimension H will be sometimes referred to as a "thickness of the sheet 111" and the third direction Z will be sometimes referred to as a "thickness direction of the sheet 111". A dimension f is the dimension between the upper surface 158 and the lower surface 157 in the troughs 162 (See Fig. 2) . A mass per unit area in the flat region 114 is the same as that in the undulating region 113 and a dimension between the upper surface 158 and the lower surface 157 in the flat region 114 is denoted by t (See Fig. 2).

When such a sheet 111 is used as the topsheet 11 in Figs. 1 to 3, the upper surface 158 and the lower surface 157 of the sheet 111 respectively become the outer surface 58 and the inner surface 57 of the topsheet 11. The undulating region 113 and the flat region 114 respectively become the central region 60a and the lateral regions 60b, and the ridges 161 and the troughs 162 respectively become the ridges 61 and the troughs 62.

In the preferred sheet 111, the dimension H is in a range of 0.6 to 2mm, the dimension f is in a range of 0.4 to 0.6mm and the dimension t is in a range of 0.2 to 0 . 7mm. These dimensions H, f, t may be measured by a method as follows. The dimension H is measured by placing a pressure plate adapted to exert a surface pressure of 3gf/cm² on a plurality of ridges 161 arranged in the first direction X, then putting a contact shoe of a dial gauge into contact with the pressure plate to obtain a measured value and subtracting a thickness of the pressure plate from a measured value. Similarly to the measuring method for the dimension H, the pressure plate may be placed on the flat region 114 and the dial gauge may be used to the dimension t. The dimension f may be measured by a method as follows. The Replacement Blade HA-100 for Cutter Knife manufactured by Kokuyo Co., LTD. is used to cut the sheet 111 in the first direction X and thereby to prepare a cut surface which is parallel to the first direction X . This cut surface is observed through Digital Microscope VHX-900 manufactured by Keyence Corporation and a photograph thereof of 25-fold magnifications is taken. Based on this photograph, a distance in the third direction Z between the short fibers forming the upper surface 158 and the short fibers forming the lower surface 157 in the troughs 162 is measured and the measured distance is obtained as the dimension f.

In the diaper 1 exemplarily illustrated in Fig. 1, the backsheet 12 of the chassis 2 is formed of a thermoplastic synthetic resin film, for example, of polyethylene having a thickness in a range of 0.01 to 0.05mm and the outer sheet 14 is formed of a nonwoven fabric such as, for example, a spunbonded nonwoven fabric, a melt blown nonwoven fabric or a spun laced nonwoven fabric. As material for the leakage-barriers 31, a poorly liquid-pervious or, more preferably a liquid-impervious nonwoven fabric or thermoplastic synthetic resin film may be used. As material for the front wings 3 and the rear wings 4, a nonwoven fabric or a laminate of a nonwoven fabric and a thermoplastic synthetic resin film may be used.

Fig. 5 is a diagram illustrating a part of a process for manufacturing a first nonwoven fabric 130a as material of the sheet 111 in Fig. 4 from a carded web 100 and Fig. 6 is a diagram illustrating a part of a process for manufacturing a continuous web 131 of the sheet 111 from the first nonwoven fabric 130a wherein the process illustrated in Fig. 5 is known from JP 2009-030218 A (PTL 3).

Referring to Fig. 5, the carded web 100 formed of the short fibers 112 and having a mass per unit area in a range of 10 to 60g/m² are placed on a running belt 200 serving as a supporting means and having air-permeability in the thickness direction and runs in a machine direction MD. As the running belt 200, a mesh plate having openings, for example, of 30 meshes or more may be used. In the machine direction MD, the process includes a first step 901 which is a pre-processing step adapted to compress the carded web 100 in its thickness direction to fuse the short fibers 112 (See Fig. 3) together and thereby to stabilize formation of the carded web 100 and a second step 902 adapted to form a pre-processed carded web 100a with the ridges 161 and the troughs 162. In the first step 901, the carded web 100 is subjected to ejection of heated first air jets 911 from a first nozzle array 910 and the first air jets 911 are sucked by a first suction box 912 through the carded web 100 and the belt 200. Airflow rate of the first air jets 911 is regulated to a level comparable to or a level slightly larger than an intake volume of the first suction box 912 so that such a pre-processing may be implemented. Temperature of the first air jets 911 is set to a temperature at which the short fibers 112 may be fused together.

In the second step 902, the pre-processed carded web 100a having been preliminarily processed in the first step 901 is subjected to ejection of a plurality of heated second air jets 921 from a second nozzle array 920 including a plurality of nozzles (not shown) arranged in a cross direction CD at a center-to-center distance a and thereby the first nonwoven fabric 130a is obtained. The second air jets 921 partially migrate the short fibers 112 in the pre-processed carded web 100a having its formation stabilized in the first step 901 in the cross direction CD so that the pre-processed carded web 100amaybe formed along portions corresponding to spaces defined between respective pairs of the adjacent second air jets 921 with the ridges 161. To achieve this, airflow rate from the second nozzle array 920 is preferably set to be larger than intake volume of the second suction box 925. In the second step 902, respective positions of the second air jets 921 arranged at the center-to-center distances a in the cross direction CD correspond to positions of the troughs 7 in the nonwoven fabric 1 illustrated in Fig. 4. In the first nonwoven fabric 130a, some portions of the short fibers 112 lying immediately below the respective second air jets 921 migrate in even shares toward both sides in the cross direction CD so as to participate in formation of the ridges 161 while the short fibers 121 remaining immediately below the second air jets 921 form the respective troughs 162. Though not illustrated, the second step 902 may include a heat treatment chamber downstream of the second nozzle array 920. In this heat treatment chamber, the first nonwoven fabric 130a may be heated so that surfaces of the respective short fibers 112 may be slightly molten so as to increase the number of regions in which the short fibers 112 in the first nonwoven fabric 130a are fused together and thereby to stabilize the formation of the first nonwoven fabric 130a.

In a third step 903 illustrated in Fig. 6, a second nonwoven fabric 130b cut from the first nonwoven fabric 130a and a desired dimension (width) in the cross direction CD is heat-pressure treated by a pressure roll 140 to obtain the continuous web 131 of the sheet 111. The pressure roll 140 includes an upper roll 141 and a lower roll 142 rotating in the machine direction MD. The upper roll 141 has diameter-enlarged sections 143 on both end sections in the cross direction CD and these diameter-enlarged sections 143 are heated at a desired temperature. The lower roll 142 supports the second nonwoven fabric 130b from below as viewed in Fig. 6. The diameter-enlarged sections 143 of the upper roll 141 cooperate with the lower roll 142 to compress opposite lateral regions 136 in the cross direction CD of the second nonwoven fabric 130b under heating effect. In this regard, the lower roll 142 may be used in a state heated to a desired temperature and may be also used in a state not heated. A clearance S between the diameter-enlarged sections 143 and the lower roll 142 is adjusted so that the opposite lateral regions 136 of the second nonwoven fabric 130b may be compressed to a desired thickness. A peripheral velocity of the diameter-enlarged sections 143 and the lower roll 142 is also adjusted so that a time required to compress the opposite lateral regions 136 to the desired thickness may be assured. The second nonwoven fabric 130b may be compressed by the diameter-enlarged sections 143 heated at a temperature 3 to 20°C lower than the melting temperature of the thermoplastic synthetic resin forming the short fibers 112 and then rapidly cooled to the room temperature. In this way, the compressed and deformed short fibers 112 may be maintained in such a deformed state and the opposite laterals 136 may be changed to the thinner flat regions 114 (See Fig. 3) having a thickness t (See Fig. 2) as a result of disappearance of the ridges 161. The second nonwoven fabric 130b having the opposite lateral regions 136 having been changed in this manner corresponds to the continuous web of the sheet 111. Specifically, this continuous web may be cut into individual sheets having a desired dimension in the machine direction MD and thereby to obtain the individual sheets 111 used as the topsheet 11 of the diaper 1. Depending on various factors such as the nature of the carded web 100 and the operating condition in the first and second steps 901, 902 illustrated in Fig. 5, generally there is a tendency such that a density in one of the ridges 161 and the troughs 162 becomes higher than a density in the other. The short fibers 112 are apt to be oriented upward in the ridges 161. In the troughs 162, there is a tendency such that the number of the short fibers 112 per unit area of the sheet 111 is relatively low and the fiber interstices are correspondingly enlarged. In the third step 903 illustrated in Fig. 6, it is possible to set up a blower downstream of the roll 140 to cool the opposite lateral regions 136 of the web 131. It is also possible to set up an additional roll similar to the roll 140 downstream of the roll 140 to assure sufficiently long time for heating and pressurizing the opposite lateral regions 136.

In the sheet 111 illustrated in Fig. 4 obtained by cutting the web 131 obtained by following the first to third steps 901 to 903, the upper surface 158 thereof in the flat region 114 is substantially flat in contrast with the upper surface 158 thereof in the undulating region 113. Consequentially, the proximal side edges 33 of the respective leakage-barriers 31 may be more reliably bonded to the topsheet 11 in the flat regions 114 than in the undulating region 113 with hot melt adhesive 32a in view of the available area for bonding and the durability of the bonding effect between the proximal side edges 33 and the topsheet 11. In the sheet 111, the density of the short fibers 112 becomes higher than that in the undulating region 113 and, in addition to this, the short fibers 112 having been apt to be oriented in the height direction, i.e., in the thickness direction changes orientation thereof so as to lie in the first direction X and the second direction Y. As an advantageous result, on the topsheet 11 of the diaper 1 illustrated in Figs. 1 and 2 formed of the sheet 111, bodily fluids flowing from the central region in the width direction B of the chassis 2 toward the lateral regions 18 in the crotch region 6, in other words, bodily fluids flowing from the central region 60a toward the lateral regions 60b of the topsheet 11 may rapidly disperse in the front-back direction A as well as in the width direction B in the opposite lateral regions 60b in which the density is relatively high and the short fibers 112 are horizontally oriented. The opposite lateral regions 18 are the regions not including the core material assembly 13 between the topsheet 11 and the backsheet 12 and therefore having no significant water-absorptive property. In view of this, bodily fluids flowing in the width direction B in the crotch region 6 may be dispersed in the lateral regions 60b of the topsheet 11 to prevent or to restrict bodily fluids from staying in the lateral regions 18 of the crotch region 6 and thereby to prevent leakage of bodily fluids from occurring in the crotch region 6. In fact, in the ridges 161 and the troughs 162 of the sheet 111, in other words, in the ridges 61 and the troughs 62 of the topsheet 11, orientation of the short fibers 112 in the height direction in the ridges 61 and relatively large interstices among the short fibers 112 in the troughs facilitate bodily fluids in the crotch region 6 to permeate the topsheet 11. However, in the ridges 61 as well as in the troughs 62, bodily fluids may not smoothly disperse in the front-back direction A as well as in the width direction B. Considering this, it is not preferable that such ridges 61 and troughs 62 define the opposite side edges 18 of the chassis 2 in the crotch region 6 because such side edges 18 may cause bodily fluids to stay in the crotch region 6. While, referring to Figs. 2 and 3, the lateral regions 60 defined by the flat portions of the topsheet 11 are partially interposed between the backsheet 12 and the proximal side edges 33 of the leakage-barriers 31, bodily fluids having been dispersed to the lateral regions 60b should not be further dispersed outward because the liquid-impervious backsheet 12 and the poorly-liquid-pervious or liquid-impervious leakage-barriers 31 are bonded together.

Fig. 7 is a diagram exemplarily illustrating the third step 903 which may be used as an alternative for the third step 903 illustrated in Fig. 6. In the step illustrated in Fig.7, the upper roll 141 of the roll 140 includes a first upper roll 141a and a second upper roll 141b having respective rotating shafts opposed to each other in the cross direction CD and these first and second upper rolls 141a, 141b heated at a desired temperature cooperate with a lower roll 142 heated as occasion arises to compress the opposite lateral regions 136 of the second nonwoven fabric 130b under heating effect. Between the first upper roll 141a and the second upper roll 141b, there is provided a hot air blower unit 146. The hot air blower unit 146 has a plurality of nozzles 147 arranged at desired pitches in the cross direction CD and hot air heated at a desired temperature is ejected from the respective nozzles 147. The second nonwoven fabric 130b corresponds to the web 100 having been treated in the first and second steps 901, 902 illustrated in Fig. 5 and, after being treated in the second step 902, the second nonwoven fabric 130b is once rolled up and sometimes stored in such a rolled up state for several weeks or even for several months. In the second nonwoven fabric 130b having been stored in the rolled up state for such a long period, the ridges 161 are compressed due to being left in such a rolled up state and sometimes deformed to substantially flat shapes. These ridges 161 may be softened by heating them at a temperature not so high to melt the short fibers 112 to recover the initial or substantially initial shapes thereof. The third step 903 illustrated in Fig. 7 is suitably used as the step to obtain the sheet 111 of the topsheets 11 from the second nonwoven fabric 130b having been stored in the rolled up state. Specifically, in the third step 903, the initial shapes of the respective ridges 161 have been deformed and substantially collapsed may be restored by heat treatment and thereby the undulating regions 113 essentially required for the sheet 111 and the flat regions 114 of the sheet 111 may be restored without compressing or, if necessary, by compressing the opposite lateral regions 136 of the second nonwoven fabric 130b or compressing them under heating effect or not as the case may be.

In the step exemplarily illustrated in Fig. 7, it is possible to replace the lower roll 142 by an endless belt (not shown) adapted to run in the machine direction MD (See Fig. 5). The endless belt may be in the form of an air-permeable mesh-type belt allowing the hot air ejected downward from the nozzle array 147 set up above the endless belt and passing through the nonwoven fabric 136b to flow further downward or in the form of an air-impervious stainless steel belt or in the form of a fabric belt adapted to reflex the hot air toward the nonwoven fabric 136b. It is also possible to exert suction effect to the hot air from underside of the air-permeable belt.

Fig. 8 is a partially cutaway perspective view of a sanitary napkin as one example of the bodily fluid absorbent wearing article according to this invention and Fig. 9 is a sectional view taken along line IX-IX in Fig. 8. The sanitary napkin 401 has a front-back direction A, a width direction B and a thickness direction C being orthogonal to each other and is shaped to be relatively long in the front-back direction A. The sanitary napkin 401 has a chassis 402 including a liquid-pervious topsheet 411, a liquid-impervious backsheet 412 and a bodily fluid absorbent core material assembly 413 interposed between these two sheets 411, 412 wherein the chassis 402 is formed along opposite side edges 418 thereof extending in parallel to each other in the front-back direction A with leakage-barriers 431, respectively. The topsheet 411 and the backsheet 412 extend outward beyond a peripheral edge 451 of the core material assembly 413 and are overlapped together outside the peripheral edge 451 of the core material assembly 413 and joined to each other with adhesive or heat sealing to define, in addition to the opposite side edges 418, opposite ends 421.

The topsheet 411 is made of the short fibers 112 exemplarily illustrated in Fig. 3 and has the central region 60a including the ridges 61 and the troughs 62 and the relatively flat lateral regions 60b in which at least the ridges 61 of the ridges 61 and the troughs 62 are in a compressed state. The boundary regions 465 defined between the central region 60a and the lateral regions 60b rectilinearly extend in the front-back direction A along the opposite side edges 418 of the peripheral edge 451 of the core material assembly 413. The backsheet 412 is formed of a thermoplastic synthetic resin film and extends outward in the width direction B beyond the opposite side edges 416 of the topsheet 411.

The leakage-barriers 431 respectively have the proximal side edges 433 bonded to the opposite side edges 418 of the chassis 402 with hot melt adhesive 32a, the opposite ends 436 fixed to the opposite ends 421 and the free side edges 437 extending in the front-back direction A in parallel to the proximal side edges 433 wherein the elastic members 439 are attached under tension to the respective free side edges 437. As will be apparent from Fig. 9, the proximal side edges 433 are bonded not only to the backsheet 412 but also to the flat lateral regions 60b in the topsheet 411 with hot melt adhesive 432a.

When such a sanitary napkin 401 is put on the wearer's body and bowed in the front-back direction A in U-shape, the free side edges 437 of the respective leakage-barriers 431 rise as indicated by imaginary lines in Fig. 9 under contraction of the elastic members 439. The proximal side edges 433 of the respective leakage-barriers 431 are bonded to the topsheet 411 in the lateral regions 60b and thereby a sufficient area for bonding may be more easily assured in comparison to the case in which the proximal side edges 433 are bonded to the topsheet 411 in the undulating regions including the ridges 61. The opposite lateral regions 60b of the topsheet 411 function in the same manner as the opposite lateral regions 60b of the topsheet 11 in the diaper 1. Specifically, bodily fluids flowing in the width direction B of the sanitary napkin 401 may be dispersed in the front-back direction A. In this invention, the arrangement of the boundary regions 465 defined between the central region 60a and the lateral regions 60b is not limited to the exemplarily illustrated arrangement such that the boundary regions 465 overlap the peripheral edge 451. For example, it is also possible to arrange the boundary regions 465 to be contiguous to the peripheral edge 451 from the outside of the peripheral edge 451, or to be contiguous to the peripheral edge 451 from the inside of the peripheral edge 451.

### {Reference Signs List}

- 1: wearing article (diaper)
- 2: chassis
- 6: crotch region
- 7: front waist region
- 8: rear waist region
- 11: topsheet
- 12: backsheet
- 13: core material assembly
- 31: leakage-barriers
- 33: side edges (proximal side edges)
- 37: side edges (free side edges)
- 51: side edges
- 57: inner surface
- 58: outer surface
- 60a: central region
- 60b: lateral regions
- 61: ridges
- 62: troughs
- A: front-back direction
- B: width direction

## Claims

1. A disposable bodily fluid absorbent wearing article (1) having a front-back direction (A) corresponding to a front-back direction of wearer's body and a width direction (B) being orthogonal to the front-back direction, including
a bodily fluid absorbent core material assembly (13) interposed between a liquid-pervious topsheet (11) and a liquid-impervious backsheet (12) in a central region as viewed in the width direction wherein
the topsheet is formed of thermoplastic synthetic resin short fibers (112) and formed on its outer surface opposed to its inner surface facing the core material assembly with ridges (61) and troughs (62) extending in the front-back direction in parallel to each other so as to undulate in the width direction and wherein
along opposite outsides in the width direction of the core material assembly, leakage-barriers (31) adapted to be elastically stretched and contracted in the front-back direction and to be spaced apart from the topsheet are formed, and
the topsheet extends outward in the width direction at least partially beyond opposite side edges of the core material assembly to define lateral regions (60b) extending outside the core material assembly, wherein:
in these lateral regions, the ridges are in a compressed state to become lower in height and to be flat;
the leakage-barriers are formed of belt-like sheet strips;
respective ones of opposite side edges of the leakage-barriers extending in the front-back direction in parallel to each other are overlapped and bonded together with the respective lateral regions of the topsheet in the width direction so that the ridges are in a compressed state in zones of the lateral regions overlapping the respective ones of the opposite side edges of the leakage-barriers; and
the ridges are in a compressed state in regions of the topsheet defined between the zones in which the respective ones of the opposite side edges of the leakage-barriers overlapped and bonded together with the lateral regions of the topsheet and the opposite side edges of the core material assembly as viewed in the width direction.

2. The wearing article defined by Claim 1, wherein the topsheet is in a compressed state also in part of a central region defined inboard of the lateral regions in the width direction to become lower in height than the ridges.

3. The wearing article defined by Claim 1 or 2, wherein the wearing article is a sanitary napkin (401).

4. The wearing article defined by any one of Claims 1 to 3, wherein the wearing article is a disposable diaper (1) including a crotch region (6) extending in the front-back direction, a front waist region (7) lying in front of the crotch region and a rear waist region (8) lying behind the crotch region and the core material assembly is contained at least in the crotch region of these regions.

5. The wearing article defined by any one of Claims 1 to 4, wherein the lateral regions of the topsheet extending in the width direction beyond the opposite side edges of the core material assembly are overlapped and bonded together with the backsheet and the respective ones of the opposite side edges of the belt-like sheet strips forming the respective leakage-barriers are overlapped and bonded together outside the respective lateral regions of the topsheet as viewed in the width direction.

6. The wearing article defined by any one of Claims 1 to 5, wherein the ridges contain the short fibers oriented in a height direction of the ridges.

## Patentansprüche

1. Einwegkleidungsstück (1) zur Absorption von Körperflüssigkeiten, das eine Vorne-Hinten-Richtung (A) hat, die mit einer Vorne-Hinten-Richtung des Körpers des Trägers korrespodiert, und einer Breitenrichtung (B), die rechtwinklig zur Vorne-Hinten-Richtung verläuft, einschließlich
ein Kernmaterialaufbau (13) für die Absorption von Körperflüssigkeiten, die zwischen einer flüssigkeitsdurchlässigen obersten Schicht (11) und einer flüssigkeitsundurchlässigen hinteren Schicht (12) in einem mittleren Bereich eingefügt wird, wenn in Breitenrichtung besichtigt, wobei
die oberste Schicht von kurzen Fasern (112) eines thermoplastischen synthetichen Harzes geformt ist, und sich an seiner Außenfläche gegenüber seiner Innenfläche des Kernmaterialaufbaus mit Erhöhungen (61) und Vertiefungen (62) in Vorne-Hinten-Richtung parallel zueinander erstreckt, sodass es sich in Breitenrichtung wellenförmig erstreckt und wobei
an gegenüberliegenden Außenteilen in Breitenrichtung des Kernmaterialaufbaus, Leckbarrieren (31) so adaptiert sind, dass sie sich in Vorne-Hinten-Richtung elastisch strecken und zusammenziehen und so auseinander von der obersten Schicht geformt sind,
die oberste Schicht sich nach Außen in Breitenrichtung wenigstens teilweise über die gegenüberliegenden Seitenränder des Kernmaterialaufbaus erstreckt, um seitliche Bereiche (60b) zu definieren, die sich außerhalb des Kernmaterialaufbaus erstrecken, wobei
in diesen seitlichen Bereichen die Erhöhungen in einem zusammengedrückten Zustand sind, um eine niedrigere Höhe zu erreichen und um flach zu sein,
die Verlustbarrieren wie riemenartige Schichtstreifen geformt sind,
jeweilige davon auf gegenüberliegenden Seitenränder der Verlustbarrieren sich in Vorne-Hinten-Richtung parallel zueinander erstrecken und überlappen und mit der jeweiligen seitlichen Bereichen der obersten Schicht in Breitenrichtung verbunden sind, sodass die Erhöhungen in Zonen der seitlichen Bereiche in einem zusammengedrückten Zustand sind, die die jeweiligen der gegenüberliegenden Seitenränder der Verlustbarrieren überlappen, und
die Erhöhungen in einem zusammengedrückten Zustand in Bereichen der obersten Schicht sind, die zwischen den Zonen definiert sind, in denen die jeweiligen der gegenüberliegenden Seitenränder der Verlustbarrieren überlappen und mit den seitlichen Bereichen des obersten Schicht verbunden und die gegenüberliegenden Seitenränder des Kernmaterialaufbaus sind, wenn in Breitenrichtung besichtigt.

2. Kleidungsstück nach Anspruch 1, wobei die oberste Schicht in einem zusammengedrückten Zustand ist, auch teilweise eines mittleren Bereichs, der innerhalb der seitlichen Bereiche in Breitenrichtung definiert ist, um eine niedrigere Höhe als die Erhöhungen zu erhalten.

3. Kleidungsstück nach Anspruch 1 oder 2, wobei das Kleidungsstück eine Binde (401) ist.

4. Kleidungsstück, nach einem der Ansprüche 1 bis 3, wobei das Kleidungsstück eine Wegwerfwindel (1) ist, die einen Schrittbereich (6), der sich in Vorne-Hinten-Richtung erstreckt, ein vorderer Taillenbereich (7), der vor dem Schrittbereich liegt, und ein hinterer Taillenbereich (8), der hinter dem Schrittbereich liegt und der Kernmaterialaufbau wenigsten im Schrittbereich dieser Bereiche gehalten wird.

5. Kleidungsstück nach einem der Ansprüche 1 bis 4, wobei sich die seitlichen Bereiche der obersten Schicht in Breitenrichtung über die gegenüberliegenden Seitenränder des Kernmaterialaufbaus erstrecken, überlappt sind, und mit der hinteren Schicht und der jeweiligen Schicht der ggegenüberliegenden Seitenränder der riemenartigen Schichtstreifen die jeweiligen Verlustbarrieren bilden, überlappt werden und außerhalb der jeweiligen seitlichen Bereiche der obersten Schicht verbunden werden, wenn in Breitenrichtung betrachtet.

6. Kleidungsstück nach einem der Ansprüche 1 bis 5, wobei die Erhöhungen die kurzen Fasern enthalten, die in Höhenrichtung der Erhöhungen angebracht sind.

## Revendications

1. Article à porter jetable (1) absorbant les fluides organiques ayant une direction avant-arrière (A) correspondant à une direction avant-arrière du corps de l'utilisateur et une direction allant dans le sens de la largeur qui est (B) perpendiculaire par rapport à la direction avant-arrière, comprenant
un ensemble de matériau central (13) absorbant les fluides organiques intercalé entre une feuille de dessus (11) perméable aux liquides et une feuille de support (12) imperméable aux liquides dans une région centrale tel que l'on peut voir dans la direction allant dans le sens de la largeur dans lequel
la feuille de dessus est formée à partir de fibres courtes (112) en résine synthétique thermoplastique et ayant, formés sur sa surface extérieure opposée à sa surface intérieure orientée vers l'ensemble de matériau central, des nervures (61) et des creux (62) s'étendant dans la direction avant-arrière de manière parallèle les uns par rapport aux autres de manière à onduler dans la direction allant dans le sens de la largeur et dans lequel
le long de parties extérieures opposées dans la direction allant dans le sens de la largeur de l'ensemble de matériau central, des barrières antifuite (31) adaptées pour être étirées et contractées de manière élastique dans la direction avant-arrière et pour être espacées par rapport à la feuille de dessus sont formées, et
la feuille de dessus s'étend vers l'extérieur dans la direction allant dans le sens de la largeur au moins partiellement au-delà de bords latéraux opposés de l'ensemble de matériau central pour définir des régions latérales (60b) s'étendant à l'extérieur de l'ensemble de matériau central, dans lequel :
dans ces régions latérales, les nervures sont dans un état comprimé pour devenir inférieures en hauteur et pour être plates ;
les barrières antifuite sont formées à partir de bandes de feuille similaires à des sangles ;
des bords latéraux respectifs de bords latéraux opposés des barrières antifuite s'étendant dans la direction avant-arrière de manière parallèle les uns par rapport aux autres sont en une relation de chevauchement et de liaison ensemble avec les régions latérales respectives de la feuille de dessus dans la direction allant dans le sens de la largeur de telle sorte que les nervures sont dans un état comprimé dans des zones des régions latérales en une relation de chevauchement avec les bords latéraux respectifs des bords latéraux opposés des barrières antifuite ; et
les nervures sont dans un état comprimé dans des régions de la feuille de dessus définies entre les zones dans lesquelles les bords latéraux respectifs des bords latéraux opposés des barrières antifuite sont en une relation de chevauchement et de liaison ensemble avec les régions latérales de la feuille de dessus et les bords latéraux opposés de l'ensemble de matériau central tel que l'on peut voir dans la direction allant dans le sens de la largeur.

2. Article à porter selon la revendication 1, dans lequel la feuille de dessus est dans un état comprimé également dans une partie de la région centrale définie vers l'intérieur des régions latérales dans la direction allant dans le sens de la largeur pour devenir inférieure en hauteur par rapport aux nervures.

3. Article à porter selon la revendication 1 ou la revendication 2, dans lequel l'article à porter est une serviette hygiénique (401).

4. Article à porter selon l'une quelconque des revendications 1 à 3, dans lequel l'article à porter est une couche jetable (1) comprenant une région au niveau de l'entrejambe (6) s'étendant dans la direction avant-arrière, une région avant au niveau de la taille (7) reposant devant la région au niveau de l'entrejambe et une région arrière au niveau de la taille (8) reposant derrière la région au niveau de l'entrejambe et l'ensemble de matériau central est contenu au moins dans la région au niveau de l'entrejambe de ces régions.

5. Article à porter selon l'une quelconque des revendications 1 à 4, dans lequel les régions latérales de la feuille de dessus s'étendant dans la direction allant dans le sens de la largeur au-delà des bords latéraux opposés de l'ensemble de matériau central sont en une relation de chevauchement et de liaison ensemble avec la feuille de support et les bords latéraux respectifs des bords latéraux opposés des bandes de feuille similaires à des sangles formant les barrières antifuite respectives et sont en une relation de chevauchement et de liaison ensemble à l'extérieur des régions latérales respectives de la feuille de dessus tel que l'on peut voir dans la direction allant dans le sens de la largeur.

6. Article à porter selon l'une quelconque des revendications 1 à 5, dans lequel les nervures contiennent les fibres courtes orientées dans une direction allant dans le sens de la hauteur des nervures.
